# EUROPEAN PATENT APPLICATION

(11) **EP 2 529 724 A1**
(43) Date of publication of application: **05.12.2012**
(21) Application number: 12181529.4
(22) Date of filing: 17.09.2008
(51) Int. Cl.: A61K 8/19, A61K 8/27, A61K 8/28, A61K 8/29, A61K 8/35, A61K 8/37, A61K 8/49, A61Q 17/04

(54) **Sunscreen compositions with low eye-sting and high SPF**

(30) Priority: 18.09.2007 US 973316 P
(62) Divisional of application: 08832609.5
(71) Applicant: MSD Consumer Care, Inc., Memphis TN 38151 (US)
(72) Inventor: Karpov, Inna, Lake Worth, FL Florida 44463 (US); Meyer, Thomas, A., Germantown, TN Tennessee 38139 (US); Wagner, John, Huntington Jr., Memphis, TN Tennessee 38117 (US); Fowler, Kevin, C., Millington, TN Tennessee 38053 (US); Beasley, Donathan, G., Memphis, TN Tennessee 38128 (US)
(74) Representative: Buchan, Gavin MacNicol

(57) **Abstract**

Sunscreen compositions that prevent, reduce or minimize eye-stinging while maintaining a high level of SPF protection.

## Description

### Field of the Invention

The present invention is generally directed to compositions for use as sunscreens, and, in particular, to sunscreen compositions that prevent, reduce, or minimize eye-stinging while maintaining a high level of SPF protection. This application claims priority from U.S. provisional patent application Serial No. 60/973,316 filed September 18, 2007.

### Background

Sunscreen compositions are applied topically to the skin to protect against damage from ultraviolet radiation (UVR). Because of the method of application, contact of the sunscreen composition with eye tissue is likely. It is well known in the art that such contact can cause undesirable eye-stinging.

The ability of a particular sunscreen composition to block UVR is generally expressed in terms of a sun protection factor (SPF). The SPF of a sunscreen composition is generally dependent upon both the type and amount of active ingredients present. In order to attain a high SPF, large amounts of active ingredients are often added. However, eye-stinging may be caused by the presence of many of these active ingredients, some of which cause eye stinging when present in large quantities, and others of which cause significant eye-stinging even when present in small quantities.

The proper mix of inactive ingredients is important in obtaining desirable properties in commercial sunscreens, such as transparency, photostability, smoothness, easy application, etc. However, the effects of inactive ingredients on eye-stinging in sunscreen compositions have been virtually ignored in the prior art. By disregarding the effects of inactive ingredients on eye-stinging, sunscreen formulators have had difficulty in preparing low eye-stinging compositions of high SPF ratings, and have been limited to preparing such sunscreen compositions with relatively low SPF ratings or have been limited in their choice of active ingredients.

### Summary

By accounting for both the positive and negative effects of inactive ingredients on eye-stinging potential, a wider variety of active ingredients, and/or an increased amount of such active ingredients, may be included in a sunscreen composition without such composition becoming excessively eye-stinging. This would be beneficial, since it would permit the preparation of a variety of low eye-stinging sunscreen compositions having high SPF ratings.

Some example embodiments of the present invention include sunscreen compositions that have high SPF ratings and low eye-sting potential by accounting for the effects of inactive ingredients.

An example embodiment of the present invention includes a sunscreen composition comprising an inorganic active ingredient selected from the group consisting of zinc oxide, titanium dioxide, iron oxide, zirconium oxide, cerium oxide, and mixtures thereof, and an organic active ingredient selected from the group consisting of octinoxate, octisalate, homosalate, avobenzone, octocrylene, para-aminobenzoic acid, cinoxate, dioxybenzone, methyl anthranilate, octocrylene, padimate O, ensulizole, sulisobenzone, trolamine salicylate, ecamsule, and mixtures thereof, wherein the composition is substantially free of oxybenzone.

In another respect, some example embodiments of the present invention include a sunscreen composition having an SPF of at least about 30.

In another respect, some example embodiments of the present invention include a sunscreen composition having an SPF of at least about 50.

In another respect, some example embodiments of the present invention inlcude a sunscreen composition having an eye-sting score of less than about 1.5.

In another respect, some example embodiments of the present invention include a sunscreen composition having an eye-sting score of less than about 1.0.

In another respect, some example embodiments of the present invention include a sunscreen composition having an eye-sting score of less than about 0.7.

In another respect, some example embodiments of the present invention include a sunscreen composition having an eye-sting score of less than about 0.5.

In another respect, some example embodiments of the present invention provides a sunscreen composition having an eye-sting score of less than about 0.3.

In another respect, some example embodiments of the present invention include a sunscreen composition comprising at least about 10% zinc oxide by weight.

In another respect, some example embodiments of the present invention include a sunscreen composition comprising at least about 15% zinc oxide by weight.

In another respect, some example embodiments of the present invention include a sunscreen composition comprising octinoxate and octisalate.

In another respect, some example embodiments of the present invention include a sunscreen composition comprising octinoxate and octisalate at weight percentages of 7.5% and 5%, respectively.

In another respect, some example embodiments of the present invention include a sunscreen composition comprising an emulsifier selected from the group consisting of silicone-based emulsifiers, glycosides, polyethylene glycols, acrylic-based emulsifiers, and mixtures thereof.

In another respect, some example embodiments of the present invention include a sunscreen composition comprising an emulsifier selected from the group consisting of Abil WE 09, Abil EM-90, DC 9011 silicone elastomer, Emulgade 68/50, Arlacel P135, Simulgel A, Simulgel EG, and mixtures thereof.

In another respect, some example embodiments of the present invention include a sunscreen composition comprising an emollient selected from the group consisting of oleaginous esters, ethers, and aloe extracts, and mixtures thereof.

In another respect, some example embodiments of the present invention include a sunscreen composition comprising an emollient selected from the group consisting of Cetiol OE, Lexol IPL, octyl palmitate, neopentyl glycol heptanoate, neopentyl glycol diheptanoate, actiphyte of aloe vera, Trivent NP-13, C₁₂-₁₅ alkyl benzoate, and mixtures thereof.

In another respect, some example embodiments of the present invention include a sunscreen composition comprising, by weight, about 15% zinc oxide, about 7.5% octinoxate, about 5% octisalate, about 5% propylene glycol, about 5% neopentyl glycol diheptanoate, about 5% C₁₂₋₁₅ alkyl benzoate, about 3% DC 9011 silicone elastomer, about 2.5% Abil EM-90, about 0.5% Germaben II, about 0.4% sodium chloride, about 0.01 % actiphyte of aloe vera, and the remainder water.

### Detailed Description of Example Embodiments

Some example embodiments of the present invention provide low eye-stinging sunscreen compositions of relatively high SPF ratings by accounting for the effects of inactive ingredients on the overall eye-stinging potential of the composition. After many years of research, the inventors have determined that a sunscreen composition's eye-stinging potential is a complex function of all of its ingredients, and inactive ingredients have important, previously overlooked, effects, which may be based upon their interaction with other, active or inactive, ingredients. Specifically, the inventors have determined that the eye-stinging potential of a particular formulation may be either enhanced or reduced depending upon the selection of inactive ingredients. Thus, by selecting appropriate combinations of active and inactive ingredients, the inventors have developed several novel low eye-stinging sunscreen compositions of high SPF ratings.

The present inventors have also found that by including a relatively large weight percentage of zinc oxide (i.e., at least about 15%) in combination with about 7.5% octinoxate and about 5% octisalate, a highly protective sunscreen composition (SPF 50) can be obtained, which simultaneously provides low eye-stinging ability. Accordingly, in some example embodiments, the composition contains at least about 15% zinc oxide, at least about 7.5% octinoxate, and at least about 5% octisalate, by weight.

The eye-stinging ability of a sunscreen composition is measured by directly instilling the composition into the eyes of human subjects. Following instillation, each subject is asked to rate the amount of perceived eye-stinging on the following scale:
0) absent
1) faint
2) mild
3) moderate
4) intense

The scores provided by each test subject are then averaged to provide the eye-stinging ability of the composition. As used in the present specification, a sunscreen composition is considered "low eye-stinging" if its score is less than about 1.0.

The term "active ingredient" is used herein to mean an ingredient in the sunscreen composition that absorbs or blocks UVR.

The term "inactive ingredient" is used herein to mean an ingredient in the sunscreen composition that does not substantially absorb or block UVR, and may include emulsifiers, emollients, preservatives, fragrances, dyes, antioxidants, chelating agents, etc.

The remaining terms used herein will be readily understood by those of ordinary skill in the art.

The sunscreen compositions of some example embodiments of the present invention provide an SPF of at least about 30 and an eye-sting score of less than about 1.5. Preferably, the sunscreen compositions provide an SPF of at least about 50 and an eye-sting score of less than about 1.0. More preferably, the eye-sting score is less than about 0.7. Even more preferably, the eye-sting score is less than about 0.5. Most preferably, the eye-sting score is less than about 0.3.

The sunscreen compositions of the present invention will now be described with reference to particularly preferred embodiments. The following embodiments are not intended to limit the scope of the invention, and it should be recognized by those skilled in the art that there are other embodiments within the scope of the claims.

The sunscreen compositions according to the present invention may be either oil in water emulsions (o/w) or water in oil emulsions (w/o), and thus may contain water and/or oil, and preferably include at least one inorganic active ingredient and at least one organic active ingredient. Suitable inorganic active ingredients include, but are not limited to, zinc oxide, titanium dioxide, iron oxide, zirconium oxide, and cerium oxide, with zinc oxide being preferred.

As used herein, "zinc oxide" may refer to either micro- or macro-sized zinc oxide, or a mixture thereof, wherein micronized (i.e., micro-sized) zinc oxide is of sufficiently small particle size so as to provide a relatively transparent composition when applied to human skin, whereas macro-sized zinc oxide provides a white composition when applied to human skin. The zinc oxide used in the sunscreen compositions of the present invention is preferably micronized zinc oxide. Preferably, the composition includes at least about 10% zinc oxide, by weight. More preferably, the composition includes at least about 15% zinc oxide, by weight.

Suitable organic active ingredients include, but are not limited to, octinoxate, octisalate, homosalate, avobenzone, octocrylene, para-aminobenzoic acid, cinoxate, dioxybenzone, methyl anthranilate, octocrylene, padimate ²O, ensulizole, sulisobenzone, trolamine salicylate, and ecamsule. Oxybenzone should not be included in the sunscreen compositions of the present invention in substantial amounts (i.e., any amount of oxybenzone included should not substantially affect the SPF or eye-sting score of the composition).

Preferably, either octinoxate or octisalate is included in the composition as an organic active ingredient. Most preferably, both octinoxate and octisalate are included in the composition as organic active ingredients. The required weight percentages of the organic active ingredients will be dependent upon the other ingredients present, both active and inactive, but are preferably of an amount necessary to provide an SPF of at least about 30, most preferably an SPF of at least about 50, and an eye-stinging score of less than about 1.5, more preferably less than about 1.0. Preferably, the sunscreen compositions of the present invention include at least about 7.5% octinoxate and at least about 5% octisalate, by weight.

Most preferably, the composition contains at least about 15% zinc oxide, at least about 7.5% octinoxate, and at least about 5% octisalate, by weight.

The sunscreen compositions of the present invention include inactive ingredients in such amounts and combinations so as to maintain eye-sting scores below about 1.5, and preferably, below about 1.0. Preferably, the inactive ingredients comprise both emulsifier(s) and emollient(s).

Suitable emulsifiers include silicone-based emulsifiers (such as polysiloxanes), glycosides, polyethylene glycols, acrylic-based emulsifiers, and mixtures thereof. Preferable emulsifiers include Abil WE 09, Abil EM-90, DC 9011 silicone elastomer, Emulgade 68/50, Arlacel P135, Simulgel A, and Simulgel EG. Particular combinations of these emulsifiers are even more preferable. For example, either Abil WE 09 or Abil EM-90 can be included in the composition along with either DC 9011 silicone elastomer, Arlacel P135, Simulgel A or Simulgel EG. Most preferable is the combination of Abil EM-90 with DC 9011 silicone elastomer.

Suitable emollients include oleaginous esters, ethers, and aloe extracts, and mixtures thereof. Preferable emollients include Cetiol OE, Lexol IPL, octyl palmitate, neopentyl glycol heptanoate, neopentyl glycol diheptanoate, actiphyte of aloe vera, Trivent NP-13, and C₁₂₋₁₅ alkyl benzoate. Most preferable is a combination of both neopentyl glycol diheptanoate, actiphyte of aloe vera, and C₁₂₋₁₅ alkyl benzoate.

The sunscreen compositions of the present invention may also include additional inactive ingredients, such as preservatives, fragrances, dyes, antioxidants, chelating agents, etc. Preferably, these additional inactive ingredients are selected so as to either reduce or minimally enhance the eye-stinging ability of the sunscreen composition.

### Examples

To more clearly describe the sunscreen compositions of the present invention, the following examples are provided. These examples are not intended to limit the scope of the invention, and one of skill in the art will understand that other compositions are within the scope of the claims.

In the following examples, eye-sting scores were determined by instilling the test sunscreen composition directly in one of each human subject's eyes and instilling in the other eye as a control either a 10% solution of Johnson & Johnson's "No More Tears" baby shampoo in water or a commercially available eye ointment (Allergan's Refresh PM Lubricant Eye Ointment). The subjects were asked to provide a score of from 0, 1, 2, 3, or 4, corresponding to absent, faint, mild, moderate, and intense eye-stinging, respectively, as described above, for the test composition. The average of the provided scores was then reported as the Eye-sting Score for each sunscreen composition.

### Examples 1-3

Three sunscreen compositions were compared to demonstrate the effects that the selection of emollients can have on eye-sting scores. In each of Examples 1-3, the sunscreen composition included zinc oxide and octinoxate as the only active ingredients, at weight percentages of 10% and 7.5%, respectively. The only difference between these compositions was in the selection of one of the emollients. Example 1 includes 3% Cromollient DP3A, Example 2 includes 3% Cetiol OE, and Example 3 includes 3% Trivent NP-13. The amounts of all of the other ingredients were equivalent, as can be seen in Table 1, where the amounts are reported by weight percentage.

**Table 1 - water in oil sunscreen compositions**

| **Ingredient** | **Example 1** | **Example 2** | **Example 3** |
|---|---|---|---|
| Magnesium Sulfate Heptahydrate | 0.7 | 0.7 | 0.7 |
| Trivent NP-13 | - | - | 3.0 |
| Zinc Oxide | 10.0 | 10.0 | 10.0 |
| Cromollient DP3A | 3.0 | - | - |
| Arlacel P-135 | 1.0 | 1.0 | 1.0 |
| Vitamin E, DL Alpha Tocopherol | 0.1 | 0.1 | 0.1 |
| Disodium EDTA | 0.01 | 0.01 | 0.01 |
| Cetiol OE | - | 3.0 | - |
| Octyl Palmitate | 8.0 | 8.0 | 8.0 |
| Abil WE 09 | 3.0 | 3.0 | 3.0 |
| Polyethylene Glycol 400 | 5.0 | 5.0 | 5.0 |
| Aloe Vera Lipo-/ Aloe Oil Extract | 0.1 | 0.1 | 0.1 |
| Jojoba Oil Colorless | 0.1 | 0.1 | 0.1 |
| Octinoxate | 7.5 | 7.5 | 7.5 |
| Germal II (Diazo lidinyl) | 0.1 | 0.1 | 0.1 |
| Water | 61.39 | 61.39 | 61.39 |
| Eye-sting Score vs. Eye Ointment Control | 0.72 | 0.45 | 0.29 |

As can be clearly seen from Table 1, inactive ingredients can have a substantial effect on the eye-stinging potential of a sunscreen composition. By substituting emollients, a 2.5 fold increase in the eye-sting score of the sunscreen composition was observed, even though the active ingredients had not been altered.

### Examples 4-6

To further illustrate the ability of inactive ingredients to substantially affect the eye-stinging ability of a sunscreen composition, three additional compositions were prepared. As reported in Table 2, each of Examples 4-6 included 10% zinc oxide and 7.5% octinoxate, by weight, as the only active ingredients, and each had an SPF of 30. Once again, the only variation among the examples was the identity and amount of inactive ingredients.

**Table 2 - oil in water sunscreen compositions**

| **Ingredient** | **Example 4** | **Example 5** | **Example 6** |
|---|---|---|---|
| Neolone 5000 | - | - | 0.01 |
| Avalure UR-450 | 4.0 | 6.0 | - |
| Emulgade 68/50 | 4.5 | 4.0 | 4.0 |
| Zinc Oxide | 10.0 | 10.0 | 10.0 |
| PNC 400 Thickening Agent | 0.3 | 0.3 | - |
| Arlacel P-135 | - | 0.3 | 0.3 |
| Lexol IPL | 5.0 | 5.0 | - |
| Dow Corn S93 Fluid R QF-13593A | 3.0 | - | - |
| Cetiol OE | - | 3.0 | 3.0 |
| Octyl Palmitate | - | - | 8.0 |
| Liquapar PE | 1.0 | 1.0 | - |
| Dow Coming 245 Fluid, 4 CST | 8.0 | - | - |
| Cetyl Dimethicone | 4.0 | 2.0 | - |
| Polyethylene Glycol 400 | - | - | 4.0 |
| Propylene Glycol | 4.0 | 4.0 | - |
| Octinoxate | 7.5 | 7.5 | 7.5 |
| USP Purified Water | 48.7 | 56.9 | 63.19 |
| Eye-sting Score vs. Eye Ointment Control | 1.35 | 1.44 | 0.44 |

As demonstrated in Table 2, varying the identities and amounts of the inactive ingredients, including emulsifiers and emollients, can greatly affect the eye-stinging ability of a sunscreen composition. Although the active ingredients remained the same, Examples 4 and 5 each display more than a 3 fold increase in eye-sting score when compared to Example 6.

### Example 7

The sunscreen composition reported in Table 3 had an SPF of 50 and eye-sting score of 0.67. By adjusting the identities and amounts of inactive ingredients, the formulation of a low eye-stinging sunscreen composition including 15% zinc oxide, 7.5% octinoxate, and 5.0% octisalate, was possible. The amounts of each component are reported by weight percent.

**Table 3 - low eye-sting sunscreen composition with SPF 50**

| **Ingredient** | **Example 7** |
|---|---|
| USP Purified Water | 51.09 |
| Zinc Oxide | 15.0 |
| Octinoxate | 7.5 |
| Octisalate | 5.0 |
| Propylene Glycol | 5.0 |
| Neopentyl Glycol Diheptanoate | 5.0 |
| C₁₂₋₁₅ Alkyl Benzoate | 5.0 |
| DC 9011 Silicone Elastomer | 3.0 |
| Abil EM-90 | 2.5 |
| Germaben II | 0.5 |
| Sodium Chloride | 0.4 |
| Actiphyte of Aloe Vera | 0.01 |
| Eye-sting Score vs. 10% J&J Solution | 0.67 |

As illustrated by the preceding examples, inactive ingredients can have a substantial influence on the eye-stinging ability of a sunscreen composition. By accounting for the effects of these inactive ingredients, a greater variety of low eye-stinging, high SPF sunscreen compositions can be prepared than previously thought possible.

Although the invention has been described with reference to particular preferred embodiments and examples, those skilled in the art will recognize that the scope of the invention is broader than those embodiments and examples contained herein.

## Claims

1. A sunscreen composition comprising an inorganic active ingredient selected from the group consisting of zinc oxide, titanium dioxide, iron oxide, zirconium oxide, cerium oxide, and mixtures thereof, and an organic active ingredient selected from the group consisting of octinoxate, octisalate, homosalate, avobenzone, octocrylene, para-aminobenzoic acid, cinoxate, dioxybenzone, methyl anthranilate, octocrylene, padimate O, ensulizole, sulisobenzone, trolamine salicylate, ecamsule, and mixtures thereof, and wherein the composition is substantially free of oxybenzone;
wherein the composition provides an SPF of at least about 30, and
wherein the composition provides an eye-sting score of less than about 1.5.

2. The sunscreen composition of claim 1 wherein the inorganic active ingredient is zinc oxide.

3. The sunscreen composition of claim 2 wherein the zinc oxide is micronized zinc oxide.

4. The sunscreen composition of claim 2 or 3, wherein the zinc oxide is present at a weight percentage of at least about 10%.

5. The sunscreen composition of claim 2, 3 or 4, wherein the organic active ingredient is octinoxate.

6. The sunscreen composition of claim 2, 3 or 4, wherein the organic active ingredient is octisalate.

7. The sunscreen composition of claim 2, 3 or 4, wherein the organic active ingredient is a mixture of octinoxate and octisalate.

8. The sunscreen composition of claim 7, further comprising a first emulsifier.

9. The sunscreen composition of claim 8, further comprising an emollient.

10. The sunscreen composition of claim 9, wherein the first emulsifier is selected from the group consisting of silicone-based emulsifiers, glycosides, polyethylene glycols, acrylic-based emulsifiers, and mixtures thereof.

11. The sunscreen composition of claim 9, wherein the emollient is selected from the group consisting of oleaginous esters, ethers, and aloe extracts, and mixtures thereof.

12. The sunscreen composition of claim 9, wherein the emollient is selected from the group consisting of Cetiol OE, Lexol IPL, octyl palmitate, neopentyl glycol heptanoate, neopentyl glycol diheptanoate, actiphyte of aloe vera, Trivent NP-13, C₁₂₋₁₅ alkyl benzoate, and mixtures thereof.

13. The sunscreen composition of claim 9 or 12, wherein the first emulsifier is selected from the group consisting of Abil WE 09, Abil EM-90, DC 9011 silicone elastomer, Emulgade 68/50, Arlacel P135, Simulgel A, Simulgel EG, and mixtures thereof.

14. The sunscreen composition of claim 13, wherein the first emulsifier is Emulgade 68/50.

15. The sunscreen composition of claim 13, wherein the first emulsifier is selected from the group consisting of Abil WE 09 and Abil EM-90, and further comprising a second emulsifier, wherein the second emulsifier is selected from the group consisting of DC 9011 silicone elastomer, Arlacel P135, Simulgel A, and Simulgel EG.

16. The sunscreen composition of claim 7, wherein the zinc oxide is present at a weight percentage of at least about 15%, the octinoxate is present at a weight percentage of at least about 7.5%, and the octisalate is present at a weight percentage of at least about 5.0%.

17. The sunscreen composition of claim 16, further comprising DC 9011 silicone elastomer and Abil EM-90.

18. The sunscreen composition of claim 16 or 17, further comprising actiphyte of aloe vera, neopentyl glycol diheptanoate, and C₁₂₋₁₅ alkyl benzoate.

19. The sunscreen composition of claim 18, further comprising water, propylene glycol, Germaben II, and sodium chloride.

20. The sunscreen composition of claim 19, wherein the composition comprises about 15% zinc oxide, about 7.5% octinoxate, about 5% octisalate, about 5% propylene glycol, about 5% neopentyl glycol diheptanoate, about 5% C₁₂₋₁₅ alkyl benzoate, about 3% DC 9011 silicone elastomer, about 2.5% Abil EM-90, about 0.5% Germaben II, about 0.4% sodium chloride, about 0.01 % actiphyte of aloe vera, and the remainder water, wherein the amounts of each component are expressed in terms of weight percentage.
